# EUROPEAN PATENT SPECIFICATION

(11) **EP 3 034 126 B1**
(45) Date of publication and mention of the grant of the patent: **26.05.2021**
(21) Application number: 15194371.9
(22) Date of filing: 12.11.2015
(51) Int. Cl.: A61M 25/09, A61M 25/01

(54) **SUBINTIMAL CROSSING WIRE GUIDE**
SUBINTIMALE KREUZENDE DRAHTFÜHRUNG
GUIDE DE FIL DE CROISEMENT SOUS-INTIMAL

(30) Priority: 13.11.2014 US 201462079241 P
(43) Date of publication of application: 22.06.2016
(73) Proprietor: Cook Medical Technologies LLC, Bloomington, IN 47404 (US)
(72) Inventor: ELSESSER, James C., Bloomington IN Indiana 47401 (US); KAY, Thomas A., Bloomington IN Indiana 47401 (US)
(74) Representative: Mathys & Squire

(56) References cited:
- EP-A2- 1 348 461
- EP-A2- 2 338 556
- WO-A1-98/18516
- WO-A1-2004/030742
- WO-A2-2007/079014
- US-A- 4 748 986
- US-A- 5 234 003
- US-A1- 2005 027 212

## Description

### BACKGROUND

The field of the present invention relates to wire guides used to advance across a lesion.

Wire guides are commonly used during angioplasties to pass through narrow passages in the body so that larger catheters and other devices may be advanced through an intraluminal passage along an already established path. Specifically, during an angioplasty, the wire guide is used to cross the portion of the intraluminal passage which is partially or completely occluded by a lesion. However, when the open passage through the lesion is extremely small or completely occluded, it can be difficult for the wire guide to cross the lesion. Furthermore, because wire guides are typically flexible to accommodate curvatures in the vasculature, they often fail to cross the lesion due to the tip of the wire guide being deflected away from the lesion or due to the body of the wire guide kinking in response to longitudinal force being exerted on the wire guide by the operator.

If a lesion is sufficiently hardened so that a wire guide cannot cross it, the wire guide may be advanced into the subintimal or endothelial layer of the blood vessel. To enter into the subintimal layer, the wire guide is advanced against the lesion until there is sufficient rigidity in the wire guide to force the wire guide into the subintimal layer. Deflected portions of the wire guide which were unable to advance across the lesion may coil in the vicinity of the proximal end of the lesion. For rigidity, wire guides typically incorporate a core with a narrow distal end and a very gradual taper, having a typical taper angle of less than 0.1 degrees, usually reaching a full diameter after 14-20 cm. Once the wire guide has entered the subintimal layer, the deflected portion of the wire guide with insufficient rigidity trails behind, doubled over. Once the wire guide has crossed the lesion and exited the subintimal layer, the wire guide must be sufficiently advanced to clear the doubled over deflected portion of the wire guide from the lesion, and then maneuvered to re-straighten the deflected portion of the wire guide so that devices may be advanced over the wire guide without interference. This process typically requires significant extra time and skill by the operator.

One problem in such an operation is that after the tip of a typical wire guide is deflected against the surface of the occlusion, it may be difficult to determine how much the wire guide must be further advanced to have sufficient rigidity to penetrate the occlusion. Additionally, the extra length of wire guide which must be advanced to cross the lesion with a typical wire guide may be problematic if the vasculature distal from the lesion is tortious or has an obstacle which prevents straightening of the doubled over proximal portion of the wire guide.

Another problem experienced during subintimal crossing with a typical wire guide with a long gradual taper is that the knuckle diameter is highly variable. This can result in the wire guide separating a greater portion of the circumference of the inner vessel layers as the wire is advanced in to the subintimal layer. In some circumstances, where the loop diameter is particularly large, the looped distal portion may wrap around the most or all of the circumference of the intraluminal passage, causing severe damage to the blood vessel as it crosses through the subintimal layer. Aside from causing additional trauma to the vessel, this high variability can decrease the ability of the wire guide to reenter the true lumen quickly once the wire guide has advanced across the lesion, due to the larger than necessary loop and therefore less concentrated force.

Another problem experienced during subintimal crossing with a typical wire guide with a long gradual taper is that, as the wire guide is advanced through the subinitimal layer, the distal portion of the wire guide will trail behind. However, because the distal portion of the wire guide is at least somewhat rigid, it will double back in a loop. The diameter of this loop is variable and could be large. As the looped distal portion is dragged through the subintimal layer, it will pass through an area of the subintimal layer equal to the loop diameter, causing excessive damage to the subintimal layer of the blood vessel.

It is desirable for a wire guide for subintimal crossing of a lesion which would be more efficient at crossing a lesion, which requires a shorter length of wire guide, which, if it forms a loop at all, forms a small diameter loop, and which results in a quicker and less complicated crossing of the lesion with minimal damage to the subintimal layer of blood vessel. It is also desirable that there would be a highly focused force on the distal portion of the wire guide to facilitate re-entry into the true lumen immediately after the wire guide has crossed the distal end of the lesion. It is also desirable that the wire guide requires only minimal or no straightening by the operator after crossing the lesion. It is further desirable that the distal portion of the wire retain a high degree of flexibility to allow the wire guide to be maneuvered through tortuous intraluminal passages.

Reference is directed to US 5,234,003 which discloses a wire guide having a generally increasing flexible tip that is resistant to fracture. The flexible tip wire guide comprises a cylindrical wire member having a substantially uniform outer diameter main portion and a distal portion with a tapered outer surface region for gradually increasing the flexibility of the tip and a proximal concave outer surface region that extends tangentially and proximally from the tapered outer surface for increasing the fracture resistance of the decreasing outer diameter distal portion. A flexible wire coil is inserted over the distal wire portion and secured to the proximal end of the distal wire portion and the distal end of the main portion with a uniform outer diameter for minimizing potential trauma to vessel walls. The outer diameter of the flexible wire coil is substantially equivalent to the outer diameter of the main portion of the wire guide to likewise minimize trauma to vessel walls. A cylindrical outer surface extends distally from the tapered outer surface of the distal wire portion to add uniform flexibility to the flexible tip for use, e.g., in a J-tip configuration.

Reference is further directed to WO 98/18516 which discloses a guidewire having a flexible distal end region that permits axisymmetric steering and forming, said guidewire comprising: a core wire having a main body and a flexible distal end at said flexible distal end region of said guidewire; a flexible first coil coupled to said distal end of said core wire, said first coil having a proximal section with a first outer diameter and a distal section with a second outer diameter smaller than said first outer diameter; and a flexible second coil coupled to said first coil and positioned substantially concentrically over said distal section of said first coil; wherein said second coil is formed from a formable material capable of reaching a stress value above its yield point such that said second coil maintains a desired bend to impart a bend to the distal end of said guidewire.

Reference is further directed to US 2005/0027212 which discloses a guide wire comprising an elongate, flexible core having a proximal region, a proximal end, a distal region, and a distal end, and the distal region having a tapered portion; a plurality of wire strands wrapped helically parallel to one another and disposed on at least a portion of the tapered distal region of the core; a polymer tie layer disposed on at least a portion of the plurality of wire strands; and a lubricious polymer layer disposed on the polymer tie layer. Furthermore patent publication WO 2004/030742 A1 discloses a medical device having a support member disposed between an elongate shaft and a coil. The support member is adapted and configured to provide structural support to the coil so as to help maintain the position of the coil relative to the shaft.

### SUMMARY

The present invention is set forth in the appended claims. A specialized wire guide may be utilized to cross a lesion through the subintimal layer, requiring a shorter length of wire guide when passing through the subintimal layer of the lumen of the vessel. The wire guide comprises an inner elongated member, an outer element, and a distal tip. The inner elongated member comprises a larger diameter proximal portion, a smaller diameter distal portion and a tapered portion between the proximal and distal portions. The tapered portion comprises a concave contour between the larger and smaller diameters, allowing the distal portion to maintain high flexibility, while the proximal portion is more rigid. The inner elongated member is surrounded by an outer element, which may comprise a polymer shell or a coil. A distal tip is coupled to the outer element at the distal end of the wire guide. The distal tip may be free of direct connection to the inner elongated member.

The wire guide is used by advancing it against a lesion, where, if the lesion is too hardened for the wire guide to pass through, the wire guide is likely to be deflected to the region where the lesion contacts the wall of the intraluminal passage. As the wire guide is subsequently advanced, the distal portion of the wire guide may be deflected. In response, the tapered portion bends allowing the distal portion to deflect, while also directing the proximal portion of the wire guide towards the subintimal layer of the intraluminal passage. After the wire guide crosses the lesion through the subintimal layer, the wire guide must be further advanced until the deflected distal portion also crosses the intraluminal passage. The short distal portion ensures that the wire guide must be advanced less than prior art wire guides. Once the deflected distal portion has resumed its original orientation with respect to the wire guide, additional devices, such as balloon catheters or sheathed stents may be advanced to the lesion over the wire guide. These devices may be used to press the lesion against an opposing side of the intraluminal passage, clearing a channel for blood flow.

### BRIEF DESCRIPTION OF SEVERAL VIEWS OF THE DRAWINGS

The invention may be more fully understood by reading the following description in conjunction with the drawings, in which:
FIG. 1 is a cross-sectional side view of a wire guide showing a distal portion of the wire guide.
FIG. 2 is a cross-sectional side view of an alternative embodiment of the wire guide showing a distal portion of the wire guide.
FIGS. 3A-3D are cross-sectional side views of a wire guide system within an intraluminal passage, showing a wire guide crossing a lesion, along with a catheter and a stent.

### DETAILED DESCRIPTION

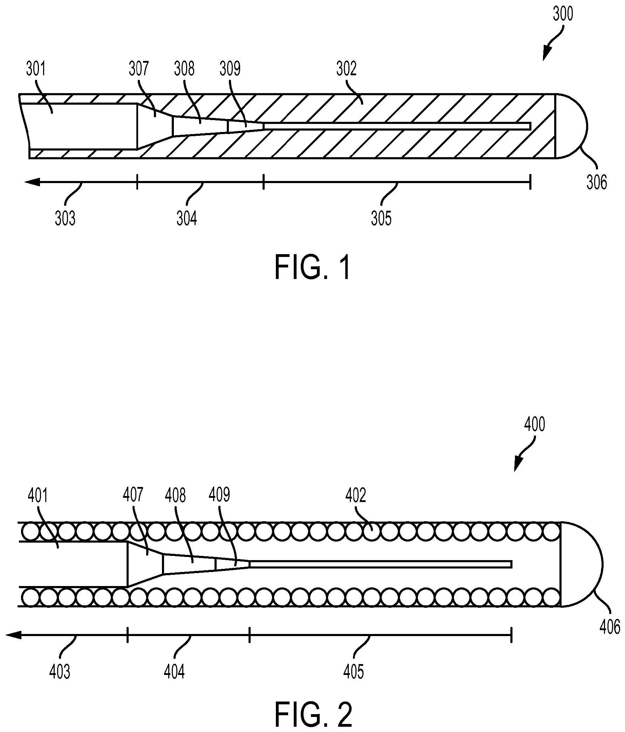

Referring to FIG. 1, the distal portion 305 of a wire guide 300 for subintimal crossing of a lesion 501 is shown. The wire guide 300 comprises an inner elongated member 301 and an outer element 302 which surrounds the inner elongated member 301. The inner elongated member 301 may be more rigid than the outer element 302 so that the diameter of the inner elongated member 301 at any point in the wire guide 300 is the primary factor contributing to the rigidity of the wire guide 300. As the diameter of the inner elongated member decreases distally, the wire guide 300 becomes more flexible closer to the distal portion 305.

The inner elongated member 301 of the wire guide 300 comprises three portions, a proximal portion 303 having a larger, substantially constant diameter, a distal portion 305 having a smaller, substantially constant diameter, and a tapered portion 304 which tapers from the larger diameter on its proximal end to the smaller diameter on its distal end.

The diameter of the proximal portion 303 of the inner elongated member 301, in part, defines the rigidity of the wire guide 300 as it passes through a subintimal layer 502 while crossing a lesion 501. By contrast, the distal portion 305 of the inner elongated member 301 defines, in part, the flexibility of the wire guide 300 as it passes through tortuous intraluminal passages 500. The distal tip 306 of the wire guide 300 may deflect against the proximal side of the lesion 501, but as the tapered portion 304 and eventually the proximal portion 303 having larger diameters and greater rigidity press against the proximal portion of the lesion 501, the wire guide 300 will eventually puncture through the lesion 501 or force a path around the lesion 501 through the subintimal layer 502. Once the wire guide 300 has been forced into the subintimal layer 502, the distal portion 305 of the wire guide 300 is sufficiently flexible to provide minimal resistance as the wire guide 300 advances across the lesion 501. The distal portion 305 may be sufficiently flexible to double over the wire guide 300 while passing through the subintimal layer 502. The tapered portion 304 and the distal portion 305 preferably have sufficient internal resistance that once it has advanced across the lesion 501, it is capable of straightening out once the doubled over portion reenters the intraluminal passage 500.

The tapered portion 304 provides a transition between the larger, more rigid proximal portion 303 and the smaller, more flexible distal portion 305. Preferably, the tapered portion 304 is configured in such a way that it distributes stress on the tapered portion 304 from bending of the distal portion 305 and minimizes the possibility of a crack or breakage between the distal portion 305 and the tapered portion 304 resulting from the resistance of passing through the subintimal layer 502. However, the configuration of the tapered portion 304 should still allow the distal portion 305 to have a high degree of flexibility, higher than the tapered portion 304 and the proximal portion 303. It is preferable to accomplish this by having the tapered portion 304 comprising a plurality of diminishing portions 307, 308, 309, where the taper angle defines the rate at which the diameter of the inner elongated member 301 decreases distally. From the proximal end to the distal end of the tapered portion 304, the first diminishing portion 307 has a first taper angle, and each distal diminishing portion 308, 309 has a corresponding taper angle which is less than the taper angle of any proximal diminishing portion. This organization of diminishing portions 307, 308, 309 creates a tapered portion 304 having a concave curvature profile.

For example, the embodiment shown in FIG. 1 comprises a tapered portion 304 with three diminishing portions 307, 308, 309. In this embodiment, the first diminishing portion 307 has a first taper angle, the second diminishing portion 308 has a second taper angle which is less than the first taper angle, and the third diminishing portion 309 has third taper angle which is less than the second taper angle. By this arrangement, the strain on the distal portion 305 of the inner elongated member 301 is distributed evenly and gradually first to the third diminishing portion 309, then to the wider second diminishing portion 308, to the even wider first diminishing portion 307, and then to the proximal portion 303 of the inner elongated member 301. By distributing the strain on the distal portion 305, the risk of the distal portion 305 cracking or breaking is minimized as it passes through the subintimal layer 502. However, the distal portion 305 retains high flexibility.

According to the invention, depending on the diameters of the proximal and distal portions 303, 305, the first diminishing portion 307 has a first taper angle ranging from 2.0 degrees to 6.0 degrees with respect to an axis passing through the wire guide 10. The second diminishing portion 308 has a second taper angle ranging from 0.3 degrees to 1.2 degrees with respect to the axis. The third diminishing portion 309 has a third taper angle ranging from 0.2 to 0.6 degrees with respect to the axis. It may be preferable, however, to include more than three diminishing portions to more closely approximate a curved tapered portion. Depending on the number of diminishing portions present, and the diameters of the proximal and distal portions 303, 305, the total taper angle of any plurality of diminishing portions of the tapered portion 304 preferably will be between 0.7 and 2.5 degrees with respect to the axis.

Typical lengths for the tapered portion 304 may be between 0.4 cm and 2.2 cm. Typical lengths for the first diminishing portion 307 may be between 0.1 cm and 0.6 cm. Typical lengths for the second diminishing portion 308 may be between 0.2 cm and 1.0 cm. Typical lengths for the third diminishing portion 309 may be between 0.1 cm and 0.6 cm. However, the lengths of each of these portions may be longer or shorter depending on design considerations including but not limited to the number of diminishing portions 307, 308, 309 in the tapered portion 304.

The length of the distal portion 305 of the inner elongated member 301 may vary depending on the embodiment of the wire guide 300, however, distal portion's 305 length may affect the functionality of the wire guide 300 in crossing a lesion 501 through the subintimal layer 502. Preferably, the distal portion 305 will be longer then the tapered portion 304 to allow for sufficient steerability of the wire guide 300 with the more flexible distal end. Preferably, though, the distal portion 305 should be significantly shorter than the proximal portion 303 which is in the intraluminal passage 500. Because the distal portion 305 is more flexible, it will provide little to no resistance when crossing the lesion 501 through the subintimal layer 502, and may double over as the proximal portion 303 proceeds through the subintimal layer 502. Typical lengths of the distal portion 305 of the inner elongated member 301 may vary from 1.0 cm to 4.0 cm, but may vary shorter or longer than these lengths depending on the design parameters and the diameter of the proximal portion 303 and the distal portion 305.

The combined lengths of the tapered portion 304 and the distal portion 305 are substantially shorter than comparable portions in prior art wire guides. The advantage of this distinction is that even if the distal portion 305 is deflected against the proximal end of the lesion 501, a short distal portion 305 will minimize coiling or bunching of the wire guide 300 about the proximal end of the lesion 501. Additionally, once the proximal portion 303 has begun to advance across the lesion 501, a shorter distal portion 305 will minimize the additional force necessary to push the doubled over distal portion 305 through area of high resistance in the vicinity of the subintimal layer 502. Furthermore, once the wire guide 300 has crossed the lesion 501 and reentered the intraluminal passage 500, a short distal portion 305 will minimize the additional length of wire guide 300 which must be advanced beyond the lesion 501 to free the distal portion 305 from the lesion 501.

From the distal tip of the inner elongated member 301 to the proximal portion 303, the inner elongated member 301 reaches its full diameter in a length between 1.4 cm and 6.2 cm. This length, however, may change depending on the design requirements for the wire guide 300, including the desired maximum diameter of the proximal portion 303 of the inner elongated member 301. One metric which can be used in designing embodiments is the ratio between the combined length of the tapered portion 304 and the distal portion 305 over the diameter of the proximal portion 303. Typical diameters for the proximal portion 303 vary between 0.05 cm and 0.10 cm. As a result typical ratios between the combined length of the tapered portion 304 and the distal portion 305 over the diameter of the proximal portion 303 vary between 12 and 124. Comparatively, prior art wire guides typically have similar ratios between 200 and 300.

The outer element 302 of the wire guide 300 shown in FIG. 1 may be comprise a shell which surrounds at least a portion of the inner elongated member 301. This material may take the form of a shell of constant diameter, or an outer layer of constant thickness which has a diameter which varies to conform to the size and shape of the inner elongated member 301. For example, in the embodiment shown in FIG. 1, an outer element 302 is shown which maintains a constant diameter, but varies in thickness to conform to the size of the inner elongated member 301 through the tapered portion 304 and distal portion 305. Alternatively, the diameter of the outer element 302 may decrease distally over the distal portion 305, or the thickness of the outer element 302 may maintain a constant thickness. Preferably, the outer element 302 is comprised of a flexible polymer, such as PTFE, to allow flexibility in the wire guide 300. Additionally, the outer element 302 may have a hydrophilic coating on its outer surface to ease movement within the intraluminal passage 500.

The outer element 302 may extend distally beyond the length of the inner elongated member 301, where it is coupled to a distal tip 306. Preferably, the distal tip 306 is shaped to better direct the movement of the wire guide 300 in navigating the vasculature, and also to prevent damage to the intraluminal passage 500. The distal tip 306 may take a variety of shapes, but preferably will decrease in diameter as it extends distally. In the embodiment shown in FIG. 1 the distal tip 306 takes the form of a curved surface, however, in other situations it may be preferable for the distal tip 306 to form a point.

Referring to FIG. 2, an alternative embodiment of the wire guide 400 is shown, wherein the inner elongated member 401 is surrounded by an outer element in the form of a coil 402. This coil 402 surrounds the inner elongated member 401 and extends distally, coupled to a distal tip 406 at the distal end of the wire guide 400. In the embodiment shown in FIG. 2, the coil 402 maintains a constant diameter as it extends distally over the proximal portion 403, the tapered portion 404, and the distal portion 405 of the inner elongated member 401, however, it may be preferable in some embodiments to change the diameter of the coils 402 to conform to the changes in diameter which occur over the length of the inner elongated member 401. In such an embodiment, the diameter of the coil 402 would decrease distally over the distal end of the wire guide 400. The coil 402 may be made of any material which is rigid enough to maintain the coiled shape while still retaining a high degree of flexibility, such as nitinol or stainless steel.

An outer element in the form of a coil 402 may provide more flexibility to the wire guide 400 while traversing tortuous vasculature. Increased flexibility, particularly in the distal end of the wire guide 400, may allow a tapered portion 404 which has increased taper angles on the first, second, and third diminishing portions 407, 408, 409 when compared to the embodiment shown in FIG. 1. Increased taper angles will result in a shorter tapered portion 404, and will allow the distal portion 405 to double over with less resistance, while still ensuring that the distal portion 405 still straightens once it has been advanced beyond the distal end of the lesion 501.

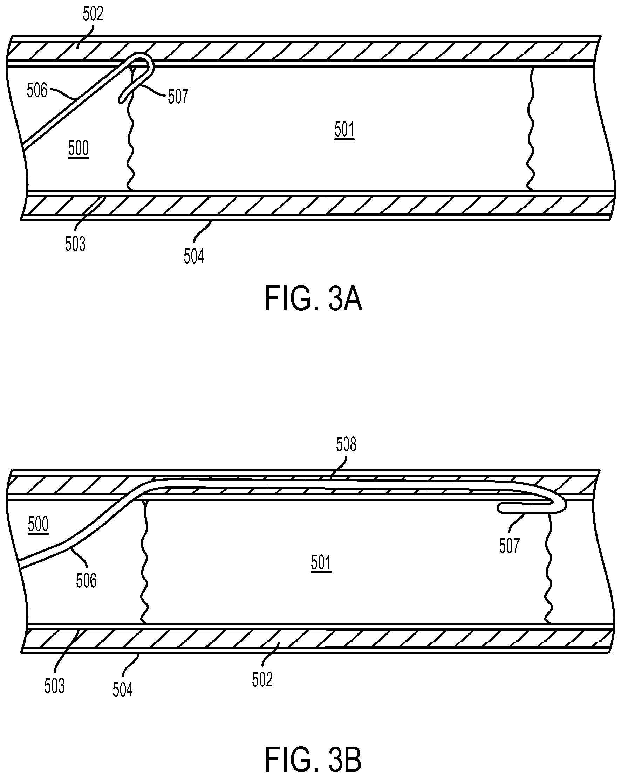

Referring to FIGS. 3A-3D, a possible procedure is shown incorporating a wire guide 506 as shown in FIGS. 1 and 2. As shown in FIG. 3A, the distal portion 507 of the wire guide 506 is pressed against the wall 503 of the intraluminal passage 500 in the vicinity of the lesion 501. As more force is applied, the flexible distal portion 507 may be deflected against the lesion 501 allowing the more rigid proximal portion to press against the lesion 501. Eventually, the proximal portion is forced through or around the proximal side of the lesion 501. If the proximal portion is forced around the lesion 501, it may enter the subintimal layer 502 of the intraluminal passage 500. The outer wall 504 of the subintimal layer 502 comprises elastic lamina and smooth muscle to which prevents the wire guide 506 from advancing through further layers of the blood vessel.

As the wire guide 506 continues to advance, the flexible distal portion 507 which was deflected on the lesion 501 may be dragged across the lesion 501 in a doubled-over position. The distal portion 507 of the wire guide will form a loop at the point where is deflects from the main body of the wire guide 506. The diameter of this loop is dependent on the rigidity of the distal portion 507 of the wire guide 506 which is determined primarily by the distal portion 305, 405 and tapered portion 304, 404 of the inner elongated member 301, 401. If the distal portion 507 of the wire guide 506 is very flexible, the loop diameter will be very small, as the majority of the distal portion 507 will be doubled-over, trailing behind the leading edge of the wire guide 506. However, if the distal portion 507 of the wire guide is more rigid, the distal portion 507 may form a larger loop while traversing the subintimal layer, though still smaller than prior art wire guides. This larger diameter loop may cause more damage to the subintimal layer of the blood vessel, however, a more rigid distal portion 507 may have the advantage of more quickly and easily reentering the intraluminal passage once the wire guide 506 has crossed the lesion 501.

The distal portion 507 of the wire guide 506 is more flexible than the rest of the wire guide 506 and thus more easily deflects as the wire guide 506 is advanced against the resistance of the lesion 501 and the subintimal layer 502. However, the remainder of the wire guide 506, comprising the tapered portion 304, 404 and proximal portion 303, 403 of the inner elongated member 301, 401 is more resistant to deflection as the wire guide 506 is advanced. As a result, only a small length of the relatively short distal portion 507 of the wire guide 506 gathers at or near the proximal end of the lesion 501, while the proximal portion of the wire guide 506 is able to press against and advance across the lesion 501. The proximal portion 303, 403 of the inner elongated member 301, 401 has sufficient rigidity due to the aprubt taper design of the tapered portion 304, 404, to prevent the proximal portion 303, 403 from bending and doubling over when the wire guide 506 is pressed against the lesion 502. The bending that results in the distal portion 507 of the wire guide 506 doubling over is restricted to the tapered portion 304, 404 and distal portion 305, 405 of the inner elongated member 301, 401, and not the proximal portion 303, 304. The bending of the tapered portion 304, 404 allows the distal portion 507 of the wire guide 506 to deflect and may also direct the proximal portion of the wire guide 506 into the subintimal layer 502.

Once a length of the proximal portion of the wire guide 506 has traversed the lesion 501, the wire guide 506 will exit the subintimal layer 502 and reenter the intraluminal passage 500 on the distal side of the lesion 501 naturally, as the resistance of advancing through the subintimal layer 502 is greater than the resistance of advancing through an unobstructed portion of the intraluminal passage 500. As shown in FIG. 3B, for most procedures to continue, the subintimal portion 508 of the wire guide 506 should be straightened, so that the distal portion 507, which may be doubled-over in the subintimal layer 502, can reenter the intraluminal passage 500 and be straightened. This straightening may be accomplished by advancing the wire guide 506 further distally into the intraluminal passage 500, so that the entire distal portion 507 is free of the subintimal layer 502. Once free, the internal resistance of the tapered portion 304 404 and the distal portion 507 should allow the distal portion to return to its original position. Alternatively, once part of the distal portion 507 of the wire guide 506 is in the intraluminal passage 500, the wire guide 506 may be retracted proximally to unwind or unbend the distal portion 507. Once the wire guide 506 has been straightened, it may be desirable to further retract or advance the wire guide 506 to prepare for larger catheters and devices to be advanced over the wire guide 506. Because the distal portion 507 is relatively short in length compared to the prior art, the wire guide 506 may only need to be advanced a shorter distance than prior art wire guides.

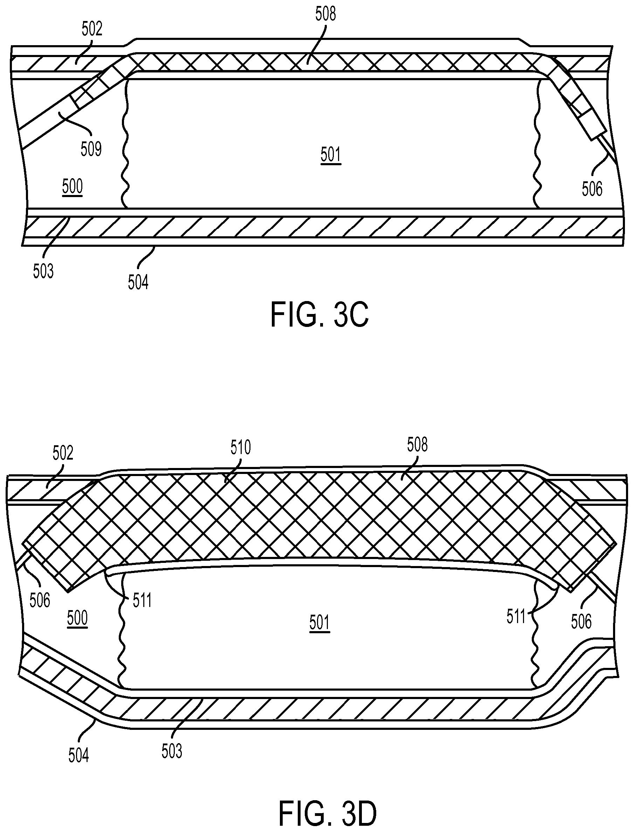

As shown in FIG. 3C, once the subintimal portion 508 is straightened, a catheter or sheath 509 containing a device 510 may be advanced through the subintimal layer 502 across the lesion 501. Devices which expand, such as balloon catheters or stents 510 are ideal for opening the intraluminal passage 500 to blood flow, but other devices may be preferable in certain circumstances. If the device is a self-expanding stent 510, for example, the catheter or sheath 509 is retracted proximally once the stent 510 is correctly positioned in the subintimal layer 502. As shown in FIG. 3D, the stent 510 expands within the subintimal layer 502 as the catheter or sheath 509 is retracted, pushing the lesion 501 against the opposing side of the intraluminal passage 500, and opening a channel for blood flow across the lesion 501 within the intraluminal passage 500. During the procedure as described above, the expansion of the stent 510, would cause at least a partial tear 511 in the wall 503 of the intraluminal passage, with a portion of the wall 503 passing between the stent 510 and the lesion 501.

Accordingly, it is now apparent that there are many advantages of the invention provided herein. In addition to the advantages that have been described, it is also possible that there are still other advantages that are not currently recognized but which may become apparent at a later time.

While preferred embodiments of the invention have been described, it should be understood that the invention is not so limited, and modifications may be made without departing from the invention. The scope of the invention is defined by the appended claims, and all devices that come within the meaning of the claims, either literally or by equivalence, are intended to be embraced by them.

## Claims

1. A wire guide, comprising:
an inner elongated member (301) having a proximal portion (303) having a first diameter, a distal portion (305) having a second diameter smaller than the first diameter, and a tapered portion arranged between the proximal portion and the distal portion, wherein the tapered portion has the first diameter on a proximal end and the second diameter on a distal end, and wherein the tapered portion comprises a contour having a proximal taper angle which is greater than a distal taper angle;
an outer element (302) which surrounds the inner elongated member; and
a distal tip coupled to the outer element;
**characterized in that** the tapered portion of the inner elongated member comprises:
a first diminishing portion (307) having a first taper angle between 2.0 degrees and 6.0 degrees,
a second diminishing portion (308) having a second taper angle between 0.3 degrees and 1.2 degrees, and
a third diminishing portion (309) having a third taper angle between 0.2 degrees and 0.6 degrees.

2. The wire guide of claim 1, wherein the outer element comprises a polymer shell.

3. The wire guide of claim 1, wherein the outer element comprises a coil about the inner elongated member.

4. The wire guide of any one of the preceding claims, wherein the diameter of the outer element decreases distally over at least a portion of the tapered portion or distal portion of the inner elongated member.

5. The wire guide of any one of claim 1 to claim 3, wherein the diameter of the outer element maintains a constant diameter over the tapered portion and distal portion of the inner elongated member.

6. The wire guide of any one of the preceding claims, wherein the distal portion has a length between 1 cm and 4 cm.

7. The wire guide of any one of the preceding claims, wherein a ratio between a combined length of the distal portion and the tapered portion over the first diameter of the proximal portion is less than 150 and preferably between 12 and 124.

8. The wire guide of any one of the preceding claims, wherein the distal tip is free of direct connection to the inner elongated member.

## Patentansprüche

1. Drahtführung, umfassend:
ein inneres längliches Glied (301), das einen proximalen Abschnitt (303) mit einem ersten Durchmesser, einen distalen Abschnitt (305) mit einem zweiten Durchmesser, der kleiner als der erste Durchmesser ist, und einen zwischen dem proximalen Abschnitt und dem distalen Abschnitt angeordneten konisch zulaufenden Abschnitt hat, wobei der konisch zulaufende Abschnitt den ersten Durchmesser an einem proximalen Ende und den zweiten Durchmesser an einem distalen Ende hat, und wobei der konisch zulaufende Abschnitt eine Kontur mit einem proximalen Konuswinkel umfasst, der größer als ein distaler Konuswinkel ist, ein äußeres Element (302), das das innere längliche Glied umgibt, und
eine an das äußere Element gekoppelte distale Spitze,
**dadurch gekennzeichnet, dass** der konisch zulaufende Abschnitt des inneren länglichen Glieds Folgendes umfasst:
einen ersten sich verkleinernden Abschnitt (307) mit einem ersten Konuswinkel zwischen 2,0 Grad und 6,0 Grad,
einen zweiten sich verkleinernden Abschnitt (308) mit einem zweiten Konuswinkel zwischen 0,3 Grad und 1,2 Grad und
einen dritten sich verkleinernden Abschnitt (309) mit einem dritten Konuswinkel zwischen 0,2 Grad und 0,6 Grad.

2. Drahtführung nach Anspruch 1, wobei das äußere Element eine Polymerschale umfasst.

3. Drahtführung nach Anspruch 1, wobei das äußere Element einen Coil um das innere längliche Glied umfasst.

4. Drahtführung nach einem der vorhergehenden Ansprüche, wobei der Durchmesser des äußeren Elements distal über mindestens einen Teil des konisch zulaufenden Abschnitts oder des distalen Abschnitts des inneren länglichen Glieds abnimmt.

5. Drahtführung nach einem der Ansprüche 1 bis 3, wobei der Durchmesser des äußeren Elements einen konstanten Durchmesser über den konisch zulaufenden Abschnitt und den distalen Abschnitt des inneren länglichen Glieds beibehält.

6. Drahtführung nach einem der vorhergehenden Ansprüche, wobei der distale Abschnitt eine Länge zwischen 1 cm und 4 cm hat.

7. Drahtführung nach einem der vorhergehenden Ansprüche, wobei ein Verhältnis zwischen einer kombinierten Länge des distalen Abschnitts und dem konisch zulaufenden Abschnitt über den ersten Durchmesser des proximalen Abschnitts kleiner als 150 und vorzugsweise zwischen 12 und 124 liegt.

8. Drahtführung nach einem der vorhergehenden Ansprüche, wobei die distale Spitze frei von direkter Verbindung zu dem inneren länglichen Glied ist.

## Revendications

1. Guide-fil, comprenant :
un élément allongé interne (301) possédant une partie proximale (303) ayant un premier diamètre, une partie distale (305) ayant un second diamètre plus petit que le premier diamètre, et une partie conique disposée entre la partie proximale et la partie distale, dans lequel la partie conique a le premier diamètre sur une extrémité proximale et le second diamètre sur une extrémité distale, et dans laquelle la partie conique comprend un contour ayant un angle de convergence proximal qui est supérieur à un angle de convergence distal ;
un élément externe (302) qui entoure l'élément allongé interne ; et
une pointe distale accouplée à l'élément externe ;
**caractérisé en ce que** la partie conique de l'élément allongé interne comprend :
une première partie décroissante (307) présentant un premier angle de convergence entre 2,0 degrés et 6,0 degrés,
une deuxième partie décroissante (308) présentant un deuxième angle de convergence entre 0,3 degré et 1,2 degré, et
une troisième partie décroissante (309) présentant un troisième angle de convergence entre 0,2 degré et 0,6 degré.

2. Guide-fil selon la revendication 1, dans lequel l'élément externe comprend une coque en polymère.

3. Guide-fil selon la revendication 1, dans lequel l'élément externe comprend une bobine autour de l'élément allongé interne.

4. Guide-fil selon l'une quelconque des revendications précédentes, dans lequel le diamètre de l'élément externe diminue distalement sur au moins une partie de la partie conique ou la partie distale de l'élément allongé interne.

5. Guide-fil selon l'une quelconque de la revendication 1 à la revendication 3, dans lequel le diamètre de l'élément externe conserve un diamètre constant sur la partie conique et la partie distale de l'élément allongé interne.

6. Guide-fil selon l'une quelconque des revendications précédentes, dans lequel la partie distale a une longueur entre 1 cm et 4 cm.

7. Guide-fil selon l'une quelconque des revendications précédentes, dans lequel un rapport entre une longueur combinée de la partie distale et de la partie conique sur le premier diamètre de la partie proximale est inférieur à 150 et de préférence entre 12 et 124.

8. Guide-fil selon l'une quelconque des revendications précédentes, dans lequel la pointe distale est dépourvue d'un raccordement direct à l'élément allongé interne.
